Europäisches Patentamt

European Patent Office

Office européen des brevets

(11) Publication number: **0 062 968 B2**

(12) **NEW EUROPEAN PATENT SPECIFICATION**

(45) Date of publication of the new patent specification: **10.07.91 Bulletin 91/28**

(51) Int. Cl.⁵: **G01N 33/50, G01N 33/53, C12N 11/02**

(21) Application number: **82301235.6**

(22) Date of filing: **11.03.82**

(54) **Support material for use in serological testing and process for the production thereof.**

(30) Priority: **18.03.81 JP 37855/81**
**31.03.81 JP 46211/81**

(43) Date of publication of application:
**20.10.82 Bulletin 82/42**

(45) Publication of the grant of the patent:
**21.11.85 Bulletin 85/47**

(45) Mention of the opposition decision:
**10.07.91 Bulletin 91/28**

(84) Designated Contracting States:
**CH DE FR GB IT LI NL**

(56) References cited:
**DE-A- 2 941 881**
**FR-A- 2 391 254**
**FR-A- 2 470 794**
**US-A- 3 966 897**
**US-A- 4 241 176**

(73) Proprietor: **FUJIREBIO KABUSHIKI KAISHA also trading as FUJIREBIO INC.**
**6-7, Shimoochiai 4-chome Shinjuku-ku Tokyo 161 (JP)**

(72) Inventor: **Ikeda, Mikio**
**7-25-23, Sunagawa-cho**
**Tachikawa-shi Tokyo (JP)**
Inventor: **Tomizawa, Takayuki**
**7-26-18-905, Shinjuku**
**Shinjuku-ku Tokyo (JP)**

(74) Representative: **Silverman, Warren et al**
**HASELTINE LAKE & CO. Hazlitt House 28 Southampton Buildings Chancery Lane London WC2A 1AT (GB)**

EP 0 062 968 B2

## Description

This invention relates to a novel support material capable of being sinsitized with an antigen and an antibody to produce a diagnostic reagent on which an enzyme can be immobilised, and to a process for the preparation thereof.

In serological testing, indirect passive haemagglutination using antigen-antibody reactions is widely employed in the diagnosis of various diseases. This reaction takes advantage of the fact that the antigen or antibody which is fixed on the surface of particles of a support material reacts with the corresponding antibody or antigen respectively in a sample serum, as a result of which, agglutination of the particles take place.

The support materials used in this method are generally particulate materials of non-biological character, such as polystryrene latex, kaolin and carbon powder, or materials of biological character such as mammalian erythrocytes and microbial cells. Generally, the particles of non-biological character which are used for the support materials are chemically stable and do not have antigenicity. However, a problem with such materials of non-biological character is that antigen and antibody are not adsorbed on them very well. For example, when the support material having antigen or antibody adsorbed thereon (sensitized carrier) is lyophilized in order to preserve it, the antigen or the antibody tends to be released from the carrier particles. Consequently, the sensitized support material needs to be chilled in the form of suspension in the substantial absence of light, and accordingly, the sensitized carrier cannot be stored over a long time. In addition, with kaolin and carbon powder of the aforementioned support materials of non-biological character, it is difficult to obtain and work with particles of equal size. Polystyrene latex is liable to undergo aggregation naturally in a neutral pH range at which indirect passive agglutination is usually carried out.

On the other hand, insofar as the use of mammalian erythrocytes and microbial cells as support materials of biological character, is concerned, sizes are almost uniform. However, their sizes depend on the species of animal from which they are obtained or the nature of the microorganism to which the cells belong and it is not always possible to have available particles having the desired size. Among the support materials of biological character, mammalian erythrocytes are most easily obtainable and their sizes are uniform. However, their surfaces possess specific antigenicity, and hence, cross reactions which give rise to non-specific agglutination may occur. Furthermore, it is difficult to obtain erythrocytes of a reproducible quality, because the biological, chemical and physical properties of erythrocytes vary according to mammalian individuals.

It has now been found that when particles of gelatin are produced from a gelatin solution containing a water-soluble polysaccharide and metaphosphate, the particles are rendered suitable for use as support material for both antigen and antibody in indirect passive agglutination by adjusting the pH of the solution to a particular pH range, and then insolubilizing the particles with an aldehyde cross-linking agent.

Gelatin microparticles insolubilized with an aldehyde cross-linking agent have been described in FR-A-2 470 794. However, the given characteristics of these microparticles would not allow their use as an erythrocyte substitute in serological testing.

Thus according to one aspect of the invention, there is provided an artificial support material for use in serological testing which is in the form of particles not more than 20 μm in diameter and having an electrophoretic mobility in an area corresponding to that of at least one type of mammalian erythrocyte which particles comprise gelatin having an isoelectric point of from pH 8 to 9, a water-soluble polysaccharide and alkali metal metaphosphate and are insolubilized with respect to water by cross-linking treatment with an aldehyde.

According to a second aspect of the invention, there is provided a process for the production of an artificial support material for use in serological testing having an electrophoretic mobility in an area corresponding to that of at least one type of mammalian erythrocyte, which comprises forming an aqueous solution containing gelatin having an isoelectric point of from pH 8 to 9, a water-soluble polysaccharide and an alkali metal metaphosphate at a temperature higher than the gelation temperature of said gelatin, adjusting the pH of said solution to a value of from 2.5 to 6.0 to produce a suspension of gelatin-containing particles which are not more than 20 μm in diameter and contacting said particles with an aldehyde cross-linking agent to render the particles insoluble in water.

According to a third aspect of the invention, there is provided a diagnostic reagent for use in serological testing, which comprises an artificial support material according to the invention which carries an antigen, an antibody or an enzyme.

The particles of the invention are produced from an aqueous solution containing gelatin, a water-soluble polysaccharide and sodium metaphosphate.

The gelatin which is used in the process of this invention may be one of a wide variety of commercial products having its isoelectric point at pH 8 to 9.

The water-soluble polysaccharide is used as a viscosity increasing agent or paste-forming agent and may be a polysaccharide as such, or a derivative or salt thereof. Examples of such polysaccharides are gum arabic,

EP 0 062 968 B2

carboxymethyl cellulose, sodium alginate and agar.

The alkali metal metaphosphate used is generally a sodium metaphosphate, i.e. a compound of the formula of $(NaPO_3)_n$, and includes sodium trimetaphosphate and sodium hexametaphosphate.

In addition, the solution may contain a water-miscible organic solvent, a nonionic or anionic surfactant, and a colouring agent.

The water-miscible organic solvent is not essential but may be added in order to accelerate the precipitation the gelatin particles. Such solvent may be an alcohol, for example methanol, ethanol, n-propanol or isopropanol, or acetone. Since the organic solvent imparts hydrophobicity to the particles, their physical and chemical properties differ from those of particles which are made without the solvent.

The anionic or the nonionic surfactant is employed in order to prevent the aggregation of particles formed in the solution. The anionic surfactant may be an alkylsulphosuccinic acid, alkylsuphomaleic acid, alkylsulphuric acid ester or polyoxyethylene alkyl ether sulphuric acid ester, and a nonionic surfactant which is used may be a polyoxyethylene fatty acid ester, a polyoxyethylene alkyl ether, a polyoxyethylene alkyl phenyl ether or a polyethylene glycol fatty acid ester. As an alternative to using this surfactant to prevent aggregation of the particles, this effect may be achieved by chilling the solution to below 10°C as soon as the particles form.

Should it be desired that the support material of the invention be coloured, a colouring agent may be added to the solution. Alternatively, the insolubilized particles which are produced may be treated with the colouring agent. However, in the former case, the colouring agent is concentrated within the particles formed in the solution, and the amount of the colouring agent used is reduced. Moreover, the colouring agent is uniformly distributed within the particles, and accordingly, the particles are uniformly and strongly coloured. Colouration of the particles may be particularly desirable when the carrier of the invention is employed for an indirect passive agglutination, when assessment of the agglutinated image of the carrier is facilitated by the colouration. The colouring agent used in the practice of this invention may be a red colourant, for example rhodamine, rose bengal, ponceau 3R, Bordeaux S, fuchsin, eosine or neutral red or a blue colourant, for example crystal violet, toluidine blue or methylene blue. Reactive dyes, such as reactive red and reactive blue are most preferred because of the permanence of colour given to the support material particles.

When carrying out the process of this invention, the concentration of gelatin in the aqueous starting solution is preferably from 0.01 to 2%, more preferably from 0.05 to 1.0% by weight, the concentration of the water-soluble polysaccharide is preferably from 0.01 to 2%, more preferably from 0.05 to 1.0% by weight, and the concentration of sodium metaphosphate is preferably from 3 to 15% by dry weight of the above gelatin. Insofar as the optional components are concerned, the preferred concentration of the water-miscible organic solvent is from 4 to 25 vol %, the preferred concentration of an anionic surfactant is from 0.001 to 0.01%, and that of a nonionic surfactant is from 0.01 to 0.1%. The preferred concentration of a colouring agent is from 0.005 to 0.5%.

There is no one essential procedure for making up the aqueous solution with the aforesaid components. For example, each component may separately be dissolved in warm water and then the solutions so produced are mixed. Alternatively, all components may be placed in a vessel, and then simultaneously dissolved in water. However, it is preferred that the water-miscible organic solvent, when used, is added later in order that good dispersion of the other components should be achieved. Moreover, the water-soluble polysaccharide is preferably dissolved separately, because this often contains a small amount of insoluble components.

At pH values lower than its isoelectric point, gelatin reacts with a water-soluble polysaccharide and a white turbidity appears. Since this white turbidity is to be avoided when carrying out the process of the invention, the turbidity must be removed by the addition of alkali prior to the pH adjustment described afterwards. Accordingly alkali is preferably added to the aqueous solution until the pH of the solution approaches the isoelectric point or even exceeds it.

The temperature of the aqueous solution is higher than the gelation temperature of the gelatin. The gelation temperature depends on the concentration of the gelatin, etc., and it is usually about 25 to 30°C. In view of the need to achieve formation of particles of adequate character, the temperature of the solution is preferably in the range of from 35 to 50°C.

Subsequently, the pH of the solution is adjusted to 2.5 to 6.0 by adding acid, usually with stirring. During the pH adjustment, particles are produced in the solution. In order to produce uniform particles, the acid is added, preferably dropwise, to the solution, while the solution is preferably heated at 35 to 50°C and stirred moderately. The optimal pH within the range of pH 2.5 to 6.0 depends on the desired size of particles and composition of the solution. For example, when the product particles are to be employed as support material for indirect passive agglutination, the particles obtained preferably have a size of from 2 to 10 μm, in which case, the optimal pH is in the range of from 4.0 to 5.5. The acid employed for the pH adjustment is not important; either an inorganic acid or an organic acid may be employed. However, a moderate acid, such as acetic acid is preferred to a strong or a weak acid.

3

No equilibrium relation exists between the particles formed by the pH adjustment and the mother liquor, and the particles do not disappear when the temperature of the solution is lowered below the gelation temperature of the gelatin. The particles are usually positively charged, although the charge which is carried depends on the ratio of gelatin to water-soluble polysaccharide, and on the surface of the particles. Metaphosphate ions are oriented and an electric double layer is constructed around the particles. This electric double layer makes the suspension of particles stable.

After the addition of the acid, the suspension of the particles may be immediately cooled below 10°C, in order to prevent the aggregation of the particles formed, as explained hereinbefore. Then, an aldehyde cross-linking agent is added to the suspension, and the particles are insolubilised. This may take place overnight, at a temperature below 10°C, if required. The amount of the cross-linking agent used may be from 0.1 to 200% by dry weight of gelatin. The cross-linking agent is for example glutaraldehyde, formaldehyde, glyoxal, crotonaldehyde, acrolein, or acetaldehyde. Glutaraldehyde is particularly preferred.

After the treatment with the cross-linking agent, the particles are recovered by for example, centrifugation, etc., and may be washed twice or three times with water preferably containing a surfactant. When a surfactant is used, it may be the same surfactant as that employed for dispersing the particles, and its concentration may also be the same.

The insolubilised particles produced by the aforesaid procedure may be used as a support material or carrier for various purposes. However, the particles sometimes swell in salt solutions. For this reason, as well as the aforesaid insolubilisation, the particles are preferably treated with further aldehyde cross-linking agent. Since the carrier may well be sensitized with antigen in a phosphate buffer solution, the support particles are preferably treated with formalin under the same conditions as are used when working with erythrocytes. The formalin treatment causes swelling of the particles to be lost, and the support particles may be kept for a long time because of the sterilizing effect of formalin.

The support material of the invention may be used to immobilise antigens, antibodies, enzymes and so on. When antigen or antibody is sensitized on the support material, the sensitization or method of immobilization may be carried out by a conventional sensitisation procedure of the type employed when using mammalian erythrocytes as support material.

The support material of the invention is almost equal in supporting ability to mammalian erythrocytes which are generally regarded as the best support material for indirect passive agglutination. Moreover, the support material of the invention is superior to mammalian erythrocytes, insofar as it is chemically and physically uniform and stable, and it has no antigenic activity. Moreover, the process of the invention enables a support material of desired particle size to be easily and inexpensively produced on a large scale.

The present invention is further illustrated by the following non-limiting examples. In the examples, all percentages are expressed on weight basis unless otherwise noted.

Example 1

(i) Preparative

4 Grams of gelatin whose isoelectric point was pH 9 were dissolved in water at 40°C, and the volume of the solution was adjusted to 100 ml. The pH of the solution was then adjusted to 9. 4 Grams of gum arabic were dissolved in water, and the volume of the solution was adjusted to 100 ml. Insoluble matter was removed by filtration, and the filtrate was warmed to 40°C.

50 ml of the above gelatin solution and 50 ml of the above filtrate were mixed, and the mixture was poured into 300 ml of 30 vol % aqueous methanol which had been previously warmed to 40°C, and the alcoholic mixture was thoroughly stirred.

1.6 ml of 10% sodium hexametaphosphate solution, 2 ml of 1% alkylsulphomaleic acid (trade name : Demol Ep, manufactured by Kao Soap Co.) and 6 ml of 1% reactive blue solution were added to the alcoholic mixture, and the mixed solution obtained was thoroughly stirred. Subsequently, 10 vol % acetic acid was added dropwise to the mixed solution while it was kept at 40°C, and the pH of the mixed solution was adjusted to 4.8. Particles were formed on the addition of acetic acid.

The suspension of the particles was cooled to 5°C in an ice bath, and 1.3 g of glutaraldehyde was added to the suspension. After stirring, the suspension was allowed to stand at 5°C overnight. Then, the suspension was centrifuged at 2000 rpm for 10 minutes, and the insolubilised particles so produced were collected. The particles were suspended in 0.005% Demol Ep solution, and then centrifuged again. This washing procedure was repeated three times, and the particles were suspended in 4 vol % formalin. The suspension was then allowed to stand at 5°C for one week.

The yield of the carrier particles so produced was 7.7 g, and 75% of the particles were in the range of 3 to

6 µm.

The electrophoretic mobility of the particles was measured using the Laser Zee system 3000 (manufactured by Pen Ken Inc., N.Y., U.S.A.). For this purpose about 1 ml of the suspension of the particles was placed in a cylindrical cell having a diameter of 1 mm and a length of 20 mm, and the mobility of the particles at 25°C at a potential gradient of 1048 V/m was measured. The electrophoretic mobility thus obtained was − 0.969 ± 0.009 µm/sec/V/cm.

Uncoloured particles were prepared in the same manner as the above coloured particles, the only procedural difference in their preparation being that reactive blue solution was not added. The electrophoretic mobility of the uncoloured particles measured under the same conditions was − 0.972 ± 0.012 µm/sec/V/cm.

Since the electrophoretic mobility of sheep erythrocytes is − 1.08 µm/sec/V/cm, the mobilities of the above particles were almost equal to that of sheep erythrocytes.


(ii) Use in TPHA test

The carrier particles produced in (i) were suspended in a phosphate buffered saline (hereinafter referred to as PBS) of pH 7.2, in a particle concentration of 2.5% the saline containing tannic acid in a concentration shown in Table 1. The suspension was raised to 37°C for 15 minutes. The particles were collected by centrifuging, and washed sufficiently with saline solution.

*Treponema palladidum* (hereinafter referred to as TP), the microbial agent responsible for syphilis, was suspended in pH 6.4 PBS, and ultrasonically treated at 10 KHz output (while held in an ice bath for 10 minutes. The thus treated TP was employed as a TP antigen.

The particles treated as aforesaid in the tannic acid solution were suspended in pH 6.4 PBS so as to be present in an amount of 5% and the suspension was mixed with an equal volume of the TP antigen solution which had previously been diluted (the dilution ratio is shown in Table 1). The mixture was warmed at 37°C for 40 minutes, and the particles became sensitized with TP antigen.

The antigen-sensitized particles were collected by centrifuging, and washed sufficiently with pH 6.4 PBS. The washed particles were then suspended in a dispersing medium so that the concentration of the particles was 5%.

The suspension of the particles sensitized with antigen thus obtained was reacted with a syphilis positive serum on the micro titre plate and the results described in Table 1 were obtained.

In a control experiment, TPHA (diagnostic reagent for syphilis, produced by Fujizoki Pharmaceutical Co. Ltd.) in which sheep erythrocytes are employed as a support material, was used. The results obtained when using both an inventive support material and the control material are shown in Table 1.

TABLE 1

| Support | Tannic acid (ppm) | Dilution ratio of antigen | Dilution ratio of serum | | | | | |
|---|---|---|---|---|---|---|---|---|
| | | | 640 | 1280 | 2560 | 5120 | 10240 | 20480 |
| | 1 | | ++ | ++ | ++ | ++ | + | − |
| | 5 | 40 | ++ | ++ | ++ | ++ | ++ | + |
| | 10 | | ++ | ++ | ++ | ++ | + | − |
| Support of the invention | 1 | | ++ | ++ | ++ | + | − | − |
| | 5 | 60 | ++ | ++ | ++ | ++ | + | − |
| | 10 | | ++ | ++ | ++ | + | − | − |
| | 1 | | ++ | ++ | + | − | − | − |
| | 5 | 80 | ++ | ++ | ++ | + | − | − |
| | 10 | | ++ | ++ | + | − | − | − |
| Sheep erythrocytes | 10 | | ++ | ++ | ++ | ++ | + | − |

(iii) Immobilization of enzyme

The support material particles obtained in part (i) of this Example were suspended in pH 7.2 PBS containing 10 ppm of tannic acid so that the concentration of the particles was 2.5%, and the suspension was warmed at 37°C for 15 minutes. The particles were collected by centrifuging, and washed thoroughly with saline solution. The washed particles were then suspended in pH 7.2 PBS in an amount of 1%.

Highly pure streptokinase was dissolved in pH 7.2 PBS so as to be present in an amount of 128 U/ml.

4 ml of the suspension of the washed particles were mixed with 4 ml of the streptokinase solution, and the mixture was warmed at 37°C for 30 minutes. The particles were collected by centrifuging, washed twice with pH 7.2 PBS, and suspended in a gelatin buffer of pH 7.2 so that the concentration of the particles was 1%. The gelatin buffer contained 5 g/litre of gelatin, 10 g/litre of sodium chloride and 13.6 g/litre of potassium dihydrogen phosphate.

The streptokinase activity of this suspension was about 60 U, and accordingly, about 12% of the enzyme in the solution was immobilised on the particles.

The streptokinase activity was determined as follows : 0.25 ml of the enzyme solution or the suspension of the particles was added to a mixture of 0.1 ml of human plasma and 0.05 ml of thrombin solution, and warmed at 37°C for 30 minutes. The enzyme activity is expressed as the maximum dilution capable of bringing fibrinolysis of coagulated plasma.

Example 2

Support particles were prepared in the same way as in Example 1, except that the 30 vol % aqueous methanol was replaced by 30 vol % aqueous ethanol, 2 ml of 1% Demol EP was replaced by 8 ml of 1 vol % polyoxyethylene phenyl ether (trade name : Emulgen A-60, manufactured by Kao Soap Co.), and the final pH achieved using acetic acid was 4.9.

The yield of particles thus obtained was 7.8 g and 70% of the particles were in the size range of 7 to 11 µm.

Example 3

Support particles were prepared in the same way as in Example 1, except that the 4 g of gum arabic was replaced by 1 g of carboxymethyl cellulose, and the 30 vol % aqueous methanol was replaced by 30 vol % aqueous ethanol. Moreover, the amounts of gelatin, 10% sodium hexametaphosphate solution, 1% Demol Ep solution, 1% reactive blue solution, and glutaraldehyde were all reduced to one quarter. The final pH using acetic acid was 4.6.

The yield of particles thus obtained was 3.8 g, and 90% of the particles were in the size range of 1 to 2 µm.

Example 4

Support particles were prepared in the same way as in Example 1, except that the volume of the gelatin solution was 40 ml, the volume of the gum arabic solution was 60 ml, the volume of the 10% sodium hexametaphosphate solution was 1.2 ml, the volume of the 1% reactive blue solution was 4.8 ml, and the weight of glutaraldehyde was 1.0 g. The final pH using acetic acid was 4.6.

The particle yield so obtained was 6.4 g, and 90% of the particles were in the range of 1 to 2 µm.

The electrophoretic mobility of the particles, measured in the same manner as in Example 1 was – 0.939 ± 0.059 µm/sec/V/cm.

Example 5

Support particles were prepared in the same way as in Example 1, except that the volume of the gelatin solution was 60 ml, the volume of the gum arabic solution was 40 ml, the volume of the 10% sodium hexametaphosphate solution was 2 ml, the volume of the 1% reactive blue solution was 7.2 ml, and the weight of glutaraldehyde was 1.8 g. The final pH using acetic acid was 4.8.

The yield of the carrier particles so obtained was 8.6 g, and 75% of the particles were in the range of 3 to 6 µm.

The electrophoretic mobility of the particles, measured in the same manner as Example 1, was – 0.996 ± 0.040 µm/sec/V/cm.

Example 6

15 Grams of gelatin having an isoelectric point of pH 9 were dissolved in 485 g of water at 40°C, and the pH of the gelatin solution was adjusted to 9 using 10% sodium hydroxide.

15 Grams of gum arabic were dissolved in 385 g of water, insoluble matter was filtered out, and the filtrate was warmed to 40°C.

60 Parts by volume of the gelatin solution were mixed with 40 parts by volume of the above filtrate, and 750 g of the mixture were poured into 2250 ml of 30 vol % aqueous ethanol which had previously been warmed to 40°C. The alcoholic mixture was then stirred thoroughly.

12 Grams of 10% sodium hexametaphosphate solution were added to the alcoholic mixture, and then 10 vol % acetic acid was added dropwise to the mixed solution until the pH of the mixed solution had reached 4.9. Particles formed as a result of the pH adjustment.

Subsequently, 7.5 g of Emulgen A-60 were added dropwise to the suspension of the particles, and the suspension was well stirred. The suspension was cooled to ambient temperature, and 0.2 g of glutaraldehyde was added. The suspension was then stirred for about 1 hour, and allowed to stand overnight. The particles thereby insolubilised were collected by centrifuging at 2000 rpm for 5 minutes. The particles were suspended in 0.2 vol % of the above surfactant solution, and then centrifuged again. This washing procedure was further repeated twice, and the washed particles were immersed in 0.05% rose bengal solution overnight. The particles, thus coloured in red, were suspended in 8% formalin, and allowed to stand at 4°C overnight.

The support particles thus obtained were almost uniform in size with most of the particles being about 10 μm in size.

The support particles were treated with tannic acid and sensitized with TP antigen, in the same way as in Example 1 (ii).

The particles sensitized with TP antigen were reacted with syphilis positive serum on the micro titre plate. The results obtained are shown in Table 2 in which are also shown the results obtained using TPHA (the product of Fujizoki Pharmaceutical Co., Ltd,) wherein sheep erythrocytes were employed as the support.

TABLE 2

| Support | Dilution ratio of serum | | | | | | | |
|---|---|---|---|---|---|---|---|---|
| | 80 | 160 | 320 | 640 | 1280 | 2560 | 5120 | 10240 |
| The Invention | + + | + + | + + | + + | + + | + + | + | − |
| Sheep Cells | + + | + + | + + | + + | + + | + + | + | − |

Example 7

10 Grams of gelatin having an isoelectric point of pH 9 were dissolved in 490 g of water at 40°C, and the pH of the gelatin solution was adjusted to 9 using 10% sodium hydroxide.

10 Grams of gum arabic were dissolved in 490 g of water, insoluble matter was filtered out, and the filtrate was warmed to 40°C.

The gelatin solution was mixed with an equal volume of the filtrate, and 750 g of the mixture obtained were poured into 2250 g of 20 vol % aqueous ethanol which had been previously warmed to 40°C. 6 Grams of 10% sodium metaphosphate were added to the alcoholic mixture thus obtained while it was stirred at 40°C. Subsequently, 10 vol % acetic acid was added dropwise to the mixed solution, until the pH of the mixed solution had reached 4.6. Particles formed as a result of this pH adjustment.

1.5 Grams of Demol Ep was gradually added to the suspension of the particles, and then the particles were insolubilised using the method of Example 6, and coloured red using 0.2% Bordeaux S solution.

The size of most of the support particles thus obtained was about 5 μm.

Example 8

15 Grams of gelatin having an isoelectric point of pH 9 were dissolved in 235 g of water at 40°C, and the gelatin solution was adjusted to pH 9 using 10% sodium hydroxide.

15 Grams of gum arabic were dissolved in 235 g of water, insoluble matter was filtered out, and the filtrate was warmed to 40°C.

The gelatin solution was mixed with the filtrate, and the mixture obtained was poured into 1500 g of 20 vol % n-propanol which had been previously warmed to 40°C. 1.2 Grams of sodium hexametaphosphate were dissolved in the alcoholic mixture, and stirred well. Subsequently, 10 vol % acetic acid was added dropwise to the mixed solution, until the pH of the mixed solution had reached 4.6. Particles having a size of about 10 μm were formed by the pH adjustment. Following the procedure of Example 6, the particles were suspended using the surfactant, insolubilised using 0.2 g of glutaraldehyde, and collected by centrifuging.

Example 9

The procedure of Example 8 was repeated, but using acetone instead of n-propanol. In this case, the final pH using acetic acid was 4.7, and the size of most of the particles obtained was about 15 μm.

Example 10

4 Grams of gelatin having an isoelectric point of pH 9 were dissolved in water at 40°C in a concentration of 4 g/dl, and the gelatin solution obtained was adjusted to pH 9 using 10% sodium hydroxide.

4 Grams of gum arabic were dissolved in water in a concentration of 4 g/dl, insoluble matter was filtered out, and the filtrate was warmed to 40°C.

50 ml of the gelatin solution were mixed with 50 ml of the filtrate, and 300 ml of 30 vol % aqueous ethanol, 1.6 ml of 10% sodium hexametaphosphate, and 6 ml of 1% reactive red solution were added to the mixture. The mixed solution was stirred well, and warmed to 40°C. Subsequently, 10 vol % acetic acid was added dropwise to the mixed solution with stirring until the pH had reached 5.0. Particles formed as a result of the pH adjustment.

The suspension of the particles was immediately cooled to 5°C in an ice bath, and 1.3 g of glutaraldehyde was added. The suspension was stirred well, and allowed to stand overnight at 4°C. The suspension was then centrifuged at 2000 rpm for 10 minutes, and the particles were collected. The collected particles were suspended in 0.01% Demol Ep solution, and centrifuged again. This washing procedure was repeated three times and the washed particles were then suspended in 4 vol % formalin and allowed to stand at 5°C for a week.

The yield of the support particles thus obtained was 6.4 g, and 75% of the particles were in the range of from 3 to 6 μm.

Example 11

5 Grams of gelatin having an isoelectric point of pH 9 were dissolved in 500 g of water at 40°C, and the gelatin solution was adjusted to pH 9 using 10% sodium hydroxide.

5 Grams of gum arabic were dissolved in 500 g of water, insoluble matter was filtered out, and the filtrate was warmed to 40°C.

The gelatin solution was mixed with the filtrate, and 4 ml of 10% sodium hexametaphosphate. Subsequently, 10 vol % acetic acid solution was added dropwise to the mixed solution with stirring until the pH had reached 4.6. Particles formed as a result of the pH adjustment.

2.5 Grams of polyoxyethylene sorbitan fatty acid ester (trade name ; Nikkol TO-10M, manufactured by Nikko Chemicals Co.) were added to the suspension of the particles and stirred well, the suspension was cooled to ambient temperature, and 0.1 g of glutaraldehyde was added to the suspension. The suspension was stirred for one hour, and allowed to stand overnight. The particles were collected by centrifuging at 2000 rpm for 10 minutes. The collected particles were suspended in 0.2 vol % of the above surfactant, and collected by centrifuging again. This washing procedure was repeated three times, and the washed particles were immersed overnight in 0.05% rose bengal solution. The red coloured beads obtained were suspended in 4% formalin, and allowed to stand for one week at 4°C.

The yield of support particles thus obtained was 18 g, and the particles, which were almost uniform in size were about 5 μm.

Example 12

Support particles were produced according to the procedure of Example 11 but varying the concentrations of gelatin and gum arabic. The results obtained are shown in Table 3. In these experiments, when the concentrations of gelatin and gum arabic were higher than 0.6%, the size of the particles suddenly became large and their size uniformity deteriorated. When the gelatin concentration was higher than 0.9%, the particles formed showed a marked tendency to aggregate all together.

TABLE 3

| Concentration of gelatin and gum arabic (%) | Sodium hexameta-phosphate (g) | Final pH | Yield (g) | Size μm |
|---|---|---|---|---|
| 0.05 | 0.04 | 3.6 | 9.0 | 5 |
| 0.1 | 0.08 | 3.8 | 12 | 5 |
| 0.25 | 0.20 | 4.1 | 15 | 5 |
| 0.4 | 0.32 | 4.5 | 17 | 5 |
| 0.5 | 0.40 | 4.6 | 17 | 5 |
| 0.6 | 0.48 | 4.7 | 18 | 20 |

Example 13

4 Grams of gelatin having an isoelectric point of pH 9 were dissolved in water at 40°C in a concentration of 4 g/dl, and the gelatin solution was adjusted to pH 9 using 10% sodium hydroxide.

4 Grams of gum arabic were dissolved in water in a concentration of 4 g/dl, insoluble matter was filtered out, and the filtrate obtained was warmed to 40°C.

50 ml of the gelatin solution were mixed with 50 ml of the filtrate, and the mixture was diluted with 300 ml of distilled water at 40°C. 1.6 ml of 10% sodium hexametaphosphate, 8 ml of 1 vol % of Emulgen A-60 solution, and 6 ml of 1% reactive blue solution were added to the diluted mixture.

Subsequently, 10 vol % acetic acid was added to the mixed solution while it was maintained at 40°C with stirring. The addition of the acetic acid was stopped when the pH of the solution had reached 4.8. Particles formed as a result of the pH adjustment.

The suspension thus obtained was cooled to 5°C in an ice bath, and 1.3 g of glutaraldehyde was added to the suspension. The suspension was then stirred well, and allowed to stand overnight at this temperature. The suspension was centrifuged at 2000 rpm for 10 minutes, and the particles were collected. The collected particles were suspended in 0.02 vol % Emulgen A-60 solution, and centrifuged again. This washing procedure was repeated three times, the washed particles were suspended in 4 vol % formalin, and allowed to stand at 5°C for a week.

The yield of support particles thus obtained was 6.2 g, and 75% of the particles were in the range of 3 to 6 μm.

Example 14

Support particles were prepared in the same way as in Example 13, except that the Emulgen A-60 solution was not added to the diluted mixture.

In this case, the yield of the support particles was 7.5 g, and 75% of the particles were in the range of 2 to 3.2 μm.

**Claims**

1. An artificial support material for use in serological testing which is in the form of particles not more than 20 μm in diameter and having an electrophoretic mobility in an area corresponding to that of at least one type of mammalian erythrocyte, which particles comprise gelatin having an isoelectric point of from pH 8 to 9, a water-soluble polysaccharide and an alkali metal metaphosphate and are insolubilised with respect to water by cross-linking treatment with an aldehyde.

2. A support material as claimed in Claim 1, wherein the water-soluble polysaccharide is gum arabic, carboxymethyl cellulose, sodium alginate or agar.

3. A support material as claimed in claims 1 or 2, wherein the metaphosphate is sodium trimetaphosphate or sodium hexametaphosphate.

4. A support material as claimed in any one of the preceding claims, wherein the aldehyde is glutaraldehyde.

5. A process for the production of an artificial support material for use in serological testing having an

9

electrophoretic mobility in an area corresponding to that of at least one type of mammalian erythrocyte, which comprises forming an aqueous solution containing gelatin having an isoelectric point of from pH 8 to 9, a water-soluble polysaccharide and an alkali metal metaphosphate at a temperature higher than the gelation temperature of said gelatin, adjusting the pH of said solution to a value of from 2.5 to 6.0 to produce a suspension of gelatin-containing particles which are not more than 20 μm in diameter and contacting said particles with an aldehyde cross-linking agent to render the particles insoluble in water.

6. A process as claimed in claim 5, wherein said solution contains :

0.01 to 2% by weight of said gelatin

0.01 to 2% by weight water soluble polysaccharide

and 3 to 15% by dry weight of the gelatin of the metaphosphate.

7. A process as claimed in Claim 5 or 6, which comprises incorporating a water-miscible organic solvent in said solution.

8. A process as claimed in Claim 7, wherein said organic solvent is methanol, ethanol, a propanol or acetone.

9. A process as claimed in Claim 7 or 8, wherein said organic solvent is present in said solution in an amount of from 4 to 25 vol %.

10. A process as claimed in any one of Claims 5 to 9, wherein an anionic or a nonionic surfactant is included in said solution.

11. A process as claimed in Claim 10, wherein said solution contains from 0.001 to 0.01% by weight of an anionic surfactant or from 0.01 to 0.1% by weight of a nonionic surfactant.

12. A process as claimed in any one of Claims 5 to 11, wherein said solution is cooled to a temperature below 10°C on effecting said pH adjustment.

13. A process as claimed in any one of Claims 5 to 12, wherein said solution additionally contains a colouring agent or said particles are contacted with a colouring agent after formation thereof.

14. A process as claimed in Claim 13, wherein said colouring agent is a reactive dye.

15. A process as claimed in Claim 13 or 14, wherein said colouring agent is present in said solution in a concentration of from 0.005 to 0.5% by weight.

16. A process as claimed in any one of Claims 5 to 15, wherein said temperature is in the range of from 35 to 50°C.

17. A process as claimed in any one of Claims 5 to 16, wherein said acid is acetic acid.

18. A process as claimed in any one of Claims 5 to 17, wherein the insolubilised particles are contacted with formalin.

19. A diagnostic reagent for use in serological testing, which comprises an artificial support material as claimed in any one of Claims 1 to 4 or produced by the process of any of Claims 5 to 18, which carries an antigen, an antibody or an enzyme.

**Patentansprüche**

1. Künstlich hergestelltes Trägermaterial zur Verwendung bei serologischen Untersuchungen in der Form von Teilchen mit einem Durchmesser von nicht mehr als 20 μm und mit einer elektrophoretischen Beweglichkeit in einem Bereich entsprechend der von wenigstens einem Säugetier-Erythrozyten-Typ, welche Teilchen Gelatine mit einem isoelektrischen Punkt von pH 8 bis 9, ein wasserlösliches Polysaccharid und ein Alkalimetallmetaphosphat enthalten und gegenüber Wasser durch eine vernetzende Aldehyd-Behandlung unlöslich gemacht sind.

2. Trägermaterial gemäß Anspruch 1, wobei das wasserlösliche Polysaccharid Gummi arabicum, Carboxymethylcellulose, Natriumalginat oder Agar ist.

3. Trägermaterial gemäß Anspruch 1 oder 2, wobei das Metaphosphat Natriumtrinetaphosphat oder Natriumhexametaphosphat ist.

4. Trägermaterial gemäß einem jeden der vorangehenden Ansprüche, wobei der Aldehyd Glutaraldehyd ist.

5. Verfahren zur Herstellung eines künstlichen Trägermaterials zur Verwendung bei serologischen Untersuchungen und mit einer elektrophoretischen Beweglichkeit in einem Bereich entsprechend der von wenigstens einem Säugetier-Erythrozyten-Typ, welches die Bildung einer wässerigen Lösung von Gelatine mit einem isoelektrischen Punkt von pH 8 bis 9, einem wasserlöslichen Polysaccharid und einem Alkalimetaphosphat bei einer Temperatur, die höher ist als die Gelierungstemperatur der Gelatine ist, die Einstellung des pH-Werts der Lösung auf eine Wert zwischen 2,5 und 6,0 zur Ausbildung einer Suspension von Gelatine enthaltenden Teilchen mit einem Durchmesser von nicht mehr als 20 μm und das Inberührungbringen der Teilchen mit einem

Aldehyd-Vernetzungsmittel zum Unlöslichmachen der Teilchen in Wasser umfaßt.

6. Verfahren gemäß Anspruch 5, wobei die besagte Lösung enthält :

0,01 bis 2 Gew.-% der besagten Gelatine,

0,01 bis 2 Gew.-% des wasserlöslichten Polysaccharids und

3 bis 15% des Metaphosphats, bezogen auf das Trockengewicht der Gelatine.

7. Verfahren gemäß Anspruch 5 oder 6, wobei der Lösung ein wassermischbares organisches Lösungsmittel zugesetzt wird.

8. Verfahren gemäß Anspruch 7, wobei das besagte organische Lösungsmittel Methanol, Ethanol, Propanol oder Aceton ist.

9. Verfahren gemäß Anspruch 7 oder 8, wobei das besagte organische Lösungsmittel in der besagten Lösung in einer Menge von 4 bis 25 Volumenprozent vorliegt.

10. Verfahren gemäß einem jeden der Ansprüche 5 bis 9, wobei die besagte Lösung eine anionisches oder nicht ionogenes oberflächenaktives Mittel enthält.

11. Verfahren gemäß Anspruch 10, wobei die besagte Lösung 0,001 bis 0,01 Gewichtsprozent eines anionischen oberflächenaktiven Mittels oder 0,01 bis 0,1 Gewichtsprozent eines nicht ionogenen oberflächenaktiven Mittels enthält.

12. Verfahren gemäß einem jeden der Ansprüche 5 bis 11, wobei die besagte Lösung auf eine Temperatur unter 10°C abgekühlt wird bei der Einstellung des besagten pH-Werts.

13. Verfahren gemäß einem jeden der Ansprüche 5 bis 12, wobei die besagte Lösung zusätzlich einen Farbstoff enthält oder die besagten Teilchen nach ihrer Bildung mit einem Farbstoff in Berührung gebracht werden.

14. Verfahren gemäß Anspruch 13, wobei der besagte Farbstoff ein Reaktivfarbstoff ist.

15. Verfahren gemäß Anspruch 13 oder 14, wobei der Farbstoff in der besagten Lösung in einer Konzentration von 0,005 bis 0,5 Gewichtsprozent vorliegt.

16. Verfahren gemäß einem jeden der Ansprüche 5 bis 15, wobei die besagte Temperatur in dem Bereich von 35 bis 50°C liegt.

17. Verfahren gemäß einem jeden der Ansprüche 5 bis 16, wobei die besagte Säure Essigsäure ist.

18. Verfahren gemäß einem jeden der Ansprüche 5 bis 17, wobei die unlöslich gemachten Teilchen mit Formalin in Berührung gebracht werden.

19. Diagnostisches Reagens zur Verwendung bei serologischen Untersuchungen, welches ein künstliches Trägermaterial gemäß einem der Ansprüche 1 bis 4 oder ein gemäß den Ansprüchen 5 bis 18 hergestelltes Produckt enthält, das ein Antigen, einen Antikörper oder ein Enzym trägt.

## Revendications

1. Matière de support artificielle destinée à être utilisée dans des tests sérologiques, qui se présente sous la forme de particules ayant un diamètre ne dépassant pas 20 µm et ayant une mobilité électrophorétique située dans un domaine correspondant à celle d'au moins un type d'érythrocytes de mammifères, lesquelles particules comprennent de la gélatine ayant un point isoélectrique de pH 8 à 9, un polysaccharide hydrosoluble et un métaphosphate de métal alcalin, et sont rendues insolubles dans l'eau par un traitement de réticulation avec un aldéhyde.

2. Matière de support selon la revendication 1, dans laquelle le polysaccharide hydrosoluble est la gomme arabique, la carboxyméthylcellulose, l'alginate de sodium ou l'agar-agar.

3. Matière de support selon la revendication 1 ou 2, dans laquelle le métaphosphate est le trimétaphosphate de sodium ou l'hexamétaphosphate de sodium.

4. Matière de support selon l'une quelconque des revendications précédentes, dans laquelle l'aldéhyde est le glutaraldéhyde.

5. Procédé de préparation d'une matière de support artificielle destinée à être utilisée dans des tests sérologiques, et ayant une mobilité électrophorétique située dans un domaine correspondant à celle d'au moins un type d'érythrocytes de mammifères, lequel procédé consiste à former une solution aqueuse contenant une gélatine ayant un point isoélectrique de pH 8 à 9, un polysaccharide hydrosoluble et un métaphosphate de métal alcalin, à une température supérieure à la température de gélification de ladite gélatine, à régler le pH de ladite solution à une valeur de 2,5 à 6,0 pour obtenir une suspension de particules contenant de la gélatine et présentant un diamètre ne dépassant pas 20 µm, et à mettre lesdites particules en contact avec un agent de réticulation aldéhyde afin de rendre les particules insolubles dans l'eau.

6. Procédé selon la revendication 5, dans lequel ladite solution contient :

0,01 à 2% en poids de ladite gélatine

0,01 à 2% en poids du polysaccharide hydrosoluble
et 3 à 15% du métaphosphate, par rapport au poids sec de la gélatine.

7. Procédé selon la revendication 5 ou 6, qui consiste à incorporer un solvant organique miscible avec l'eau dans ladite solution.

8. Procédé selon la revendication 7, dans lequel ledit solvant organique est le méthanol, l'éthanol, un propanol ou l'acétone.

9. Procédé selon la revendication 7 ou 8, dans lequel ledit solvant organique est présent dans ladite solution à raison de 4 à 25% en volume.

10. Procédé selon l'une quelconque des revendications 5 à 9, dans lequel on incorpore dans ladite solution un tensio-actif anionique ou non-ionique.

11. Procédé selon la revendication 10, dans lequel ladite solution contient de 0,001 à 0,01% en poids d'un tensio-actif anionique ou de 0,01 à 0,1% en poids d'un tensio-actif non-ionique.

12. Procédé selon l'une quelconque des revendications 5 à 11, dans lequel on refroidit ladite solution à une température inférieure à 10°C lorsqu'on effectue ledit réglage du pH.

13. Procédé selon l'une quelconque des revendications 5 à 12, dans lequel ladite solution contient en outre un agent colorant, ou bien on met en contact lesdites particules avec un agent colorant après leur formation.

14. Procédé selon la revendication 13, dans lequel ledit agent colorant est un colorant réactif.

15. Procédé selon la revendication 13 ou 14, dans lequel ledit agent colorant est présent dans ladite solution en une concentration de 0,005 à 0,5% en poids.

16. Procédé selon l'une quelconque des revendications 5 à 15, dans lequel ladite température est comprise entre 35 et 50°C.

17. Procédé selon l'une quelconque des revendications 5 à 16, dans lequel ledit acide est l'acide acétique.

18. Procédé selon l'une quelconque des revendications 5 à 17, dans lequel on met les particules insolubilisées en contact avec de la formaline.

19. Réactif diagnostique devant servir dans des tests sérologiques, qui comprend une matière de support artificielle telle que revendiquée dans l'une quelconque des revendications 1 à 4 ou préparée par le procédé de l'une quelconque des revendications 5 à 18, et qui porte un antigène, un anticorps ou une enzyme.